# EUROPEAN PATENT APPLICATION

(11) **EP 3 103 385 A1**
(43) Date of publication of application: **14.12.2016**
(21) Application number: 16173172.4
(22) Date of filing: 06.06.2016
(51) Int. Cl.: A61B 5/00, A61B 5/11, A61G 7/018, G08B 21/04, A61G 7/05, A61G 7/057, A61G 5/00, G08B 13/196

(54) **IMAGE TRANSMISSION OR RECORDING TRIGGERED BY BED EVENT**

(30) Priority: 12.06.2015 US 201514737674
(71) Applicant: Hill-Rom Services, Inc., Batesville, IN 47006-9167 (US)
(72) Inventor: HOOD, Michael, Scott, Batesville, IN 47006 (US); NEWKIRK, David C., Lawrenceburg, IN 47025 (US)
(74) Representative: Findlay, Alice Rosemary

(57) **Abstract**

A system includes a patient bed and a camera system that automatically, in response to an occurrence of a bed event, does one or more of the following: takes a picture of a patient on the bed, records a video of the patient for a period of time, or transmits a video of the patient for a period of time.

## Description

Patient beds having alarms or alerts associated with various bed events are known in the art. For example, in some patient beds, bed alarms or alerts are generated in response to lowering of a siderail of the bed, releasing a caster brake of the bed, raising an upper frame of a bed from its lowest position, movement of a patient toward exiting the bed, and movement of a head section of a mattress support deck above or below a threshold angle, just to name a few. The terms "alarm" and "alert" are used interchangeably herein and each is intended to have the meaning of both. These bed event alarms are indicative of conditions that, in some instances, relate to patient safety. However, in some circumstances, the occurrence of the bed event may not require the assistance or intervention of a caregiver.

With regard to the example of a bed exit or patient position monitoring system, there is a potential for a high number of false alarms. Such false alarms may result from a patient simply repositioning themselves in bed rather than actually moving toward exiting the bed or a visitor sitting or leaning on the bed and then removing their weight from the bed. This can lead to alert fatigue by caregivers which can potentially create a "cry-wolf phenomenon." That is, after a experiencing multiple false alarms, caregivers may be less responsive to such future alerts or may even ignore the future alerts. Setting the bed exit alarm level to the least sensitive level in an attempt to reduce false alarms does not afford caregivers sufficient time, in some instances, to reach the bedside before the patient has exited the bed and starts walking away from the bed. These circumstances can potentially lead to bed exit or patient position monitoring systems not being used at all by caregivers.

Today, some healthcare facilities may employ remote monitoring of patient rooms using camera based systems and associated monitoring personnel. However, remote monitoring personnel do not have the benefit of regularly interacting with the patient and thus, may not be as effective as bedside caregivers in determining the need to intervene. They may also be less effective in negotiating with patients to stay in bed or wait for assistance, for example, because they do not have the personal relationship with the patient that assigned caregivers do. Thus, what is needed is a system that reduces alarm fatigue of caregivers and that provides an effective patient safety enhancement.

The present disclosure provides an apparatus or system which may comprise one or more of the following features alone or in any combination.

A system may include a patient bed and a camera system that automatically, in response to an occurrence of a bed event, may record a video feed of a patient on the bed for a period of time and/or transmit a video feed of the patient for a period of time. The camera system may also take a picture of the patient.

The bed event may include a weight reading being taken, for example. The weight reading and the picture or video may be transmitted from the bed to a remote computer. Alternatively or additionally, the bed event may include movement of the patient on the bed. For example, the movement of the patient on the bed may include a threshold amount of movement that may be sufficient to trigger a patient position monitoring alarm of the patient bed. As another example, the movement of the patient on the bed may include a threshold amount of movement that may be sufficient to trigger a bed exit alarm of the patient bed. The camera system may include a camera and at least one computer device remote from the camera.

According to this disclosure, a system may include a patient bed, a first computer device that may be remote from the patient bed, a camera system that may automatically, in response to occurrence of a bed event, provide a video feed to the first computer device, and a portable computer device that may be transported by a caregiver. The video feed may be sent to the portable computer device by the first computer device in response to receipt of the video feed from the camera system.

In some embodiments, the first computer device may record the video feed for a period of time. A second computer device may be remote from the patient bed. The first computer device may transmit the video feed to the second computer device for recording and storage by the second computer device. The portable computer device may include a workstation on wheels or a wearable device. For example, the wearable device may include eye glasses with video display capability in a field of view of the caregiver or the wearable device may be configured to be worn on the caregiver's wrist. Alternatively or additionally, the portable computer device may include a handheld phone.

Alternatively or additionally, the video feed may be transmitted by the first computer device to a display of a patient bed that may be near the caregiver. The system may include a real-time locating system (RTLS) that monitors the whereabouts of caregivers and equipment in the healthcare facility. The bed to which a video feed is to be transmitted is determined based on information from the RTLS indicating which bed a particular caregiver is near. Further alternatively or additionally, the video feed may be transmitted to a display mounted to a wall of a room in which the caregiver may be located. Information from the RTLS is used in such instances to determine the room in which the caregiver is located, thereby to determine the display to which the video feed is to be transmitted.

In some embodiments, the video feed may be transmitted to a nurse station computer that may be located at a nursing station. According to this disclosure, the first computer device may be configured to control at least one of angle and zoom of a camera of the camera system. In response to the video feed being sent to the portable computer device, an audio channel may be opened between the portable computer device and a speaker in the patient's room so that the caregiver may be able to talk to the patient.

In some embodiments, a caregiver may be able to retrieve past video recordings or past pictures for viewing on a display such as a graphical user interface (GUI) of a patient bed, a wall mounted display, and/or a display of a computer device such as the caregiver's portable computer device. Such past videos or pictures may be useful, for example, in connection with weighing a patient on the patient bed or in connection with zeroing a weigh scale system of the patient bed. The caregiver may compare the past video or picture with the current state of the patient bed to check for any changes in equipment, sheets, pillows, etc. on the bed. If there are changes as compared to the past state of the bed, the caregiver may take steps to re-zero the weigh scale system so that subsequent weight readings of the associated patient may be accurate.

The invention will now be further described by way of example with reference to the accompanying drawings, in which:
Fig. 1 is a partly perspective, partly diagrammatic view showing a camera mounted to a ceiling and aimed at a patient on a hospital bed, the bed being coupled to a network via a network interface, the camera also being coupled to the network, a real time locating system (RTLS) being coupled to the network and receiving a wireless signal from a tag worn by a caregiver, and a caregiver display being coupled to the network to receive a video feed of the patient in response to the occurrence of a bed event;
Fig. 2 is a block diagram showing components of the patient bed;
Fig. 3 is a block diagram showing the camera, various examples of caregiver display devices, and various computer devices of the network that are associated with providing a video feed to one or more of the caregiver display devices;
Fig. 4 is a perspective view showing a field of view of a caregiver wearing eye glasses having video display capability and the video feed being displayed in an upper right corner of the field of view; and
Fig. 5 is a perspective view of a portion a patient bed having a footboard with a camera unit extending upwardly from a central region of a foot board, the camera unit having a camera that faces toward a patient on the bed and a GUI, the bed having control circuitry that commands the camera to take a picture and/or to record and/or transmit a video of the patient for a period of time in response to a bed event occurring.

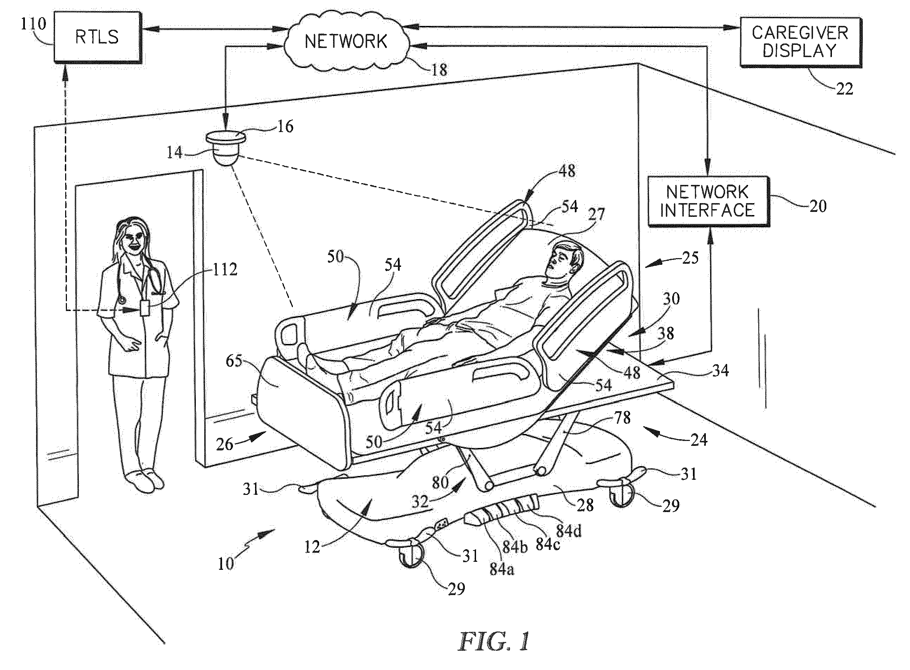

As shown in Fig. 1, a system 10 includes a patient bed 12, a camera 14 mounted to a ceiling 16, a network 18, a network interface 20, and a caregiver display 22. According to this disclosure, a picture or video feed is provided to caregiver display 22 in response to occurrence of a bed event (aka a triggering event) as detected by bed 12 or as detected by one or more computer devices of network 18. Prior to the triggering event, the picture or video feed is not provided to the caregiver display 22. Thus, patient privacy is maintained until a triggering event occurs. The triggering event is communicated from bed 12 via network interface 20 to one or more remote computer devices of network 18 and then the one or more remote computer devices provide the picture or video feed to caregiver display 22 under appropriate circumstances as will be discussed in further detail below. Bed alerts or alarms are examples of triggering events but other bed events that aren't considered

In some embodiments, the picture or video feed is recorded and stored in memory of one or more computer devices of network 18 in response to a bed event occurring. The video feed being recorded is recorded by one or more computer devices of network 18 during the same duration of time that the video feed is provided to the caregiver display 22 in some embodiments. Recording the video feed for durations greater than or less than the amount of time it is provided to the caregiver display 22 is also within the scope of this disclosure. In some embodiments, the transmission of the video feed to caregiver display 22 ceases in response to the bed event being rectified. The recording of the video feed also ceases in response to the bed event being rectified in some embodiments. The transmission and/or recording of the video feed continues for a threshold amount of time after the bed event is rectified in some embodiments, but yet is still considered to cease in response to the bed event being rectified according to this disclosure.

In Fig. 1, the diagrammatic double-headed arrows between bed 12 and network interface 20, between camera 14 and network 18, between network 18 and network interface 20, and between network 18 and caregiver display 22 are intended to be illustrative of wired and/or wireless communication connections between these devices. For example, several types of devices may embody the network interface 20 according to this disclosure. In some embodiments, network interface 20 comprises a wireless access point (WAP) or a wireless interface unit (WIU) of a nurse call system, in which case the double headed arrow between bed 12 and network interface 20 represent a wireless communication link. In other embodiments, network interface 20 comprises a bed interface unit (BIU) of a nurse call system in which case the double headed arrow between bed 12 and network interface unit 20 represents a wired communication link. See, for example, U.S. Patent Nos. 7,852,208; 7,538,659; 7,319,386 and 5,699,038 as well as U.S. Patent Application Publication Nos. 2015/0081335 A1, 2009/0217080 A1, 2009/0212925 A1 and 2009/0212926 A1 for additional information concerning connectivity of patient beds to networks including nurse call systems.

The following description discusses various components of bed 12. This description provides background to give context to the types of bed events, alarms, or occurrences that trigger video transmission and/or recording by system 10. It is contemplated that one or more computer devices of network 18 are used to select which bed events result in video transmission and/or recording and which do not. Thus, a menu of all available bed events is provided to users of system 10 on a display of a computer device and the user selects from among the overall menu of events, which ones constitute a video trigger. Thus, a bed 12 in one room may have one set of bed events selected for video triggering and another bed in another room may have a different set of bed events selected for video triggering.

Referring still to FIG. 1, a frame 25 of bed 12 includes a base 28, an upper frame assembly 30 and a lift system 32 coupling upper frame assembly 30 to base 28. Lift system 32 is operable to raise, lower, and tilt upper frame assembly 30 relative to base 28. Bed 12 has a head end 24 and a foot end 26. Bed 12 further includes a footboard 65 at the foot end 26 and a headboard (not shown) at the head end 24. Footboard 65 is coupled to upper frame assembly 30. Base 28 includes wheels or casters 29 that roll along a floor as bed 12 is moved from one location to another. A set of foot pedals 31 are coupled to base 28 and are used to brake and release casters 29.

Illustrative hospital bed 12 has four siderail assemblies coupled to upper frame assembly 30 as shown in FIG. 1. The four siderail assemblies include a pair of head siderail assemblies 48 (sometimes referred to as head rails) and a pair of foot siderail assemblies 50 (sometimes referred to as foot rails). Each of the siderail assemblies 48, 50 is movable between a raised position, as shown in FIG. 1, and a lowered position (not shown). Siderail assemblies 48, 50 are sometimes referred to herein as siderails 48, 50. Each siderail 48, 50 includes a barrier panel 54 and a linkage (not shown). Each linkage is coupled to the upper frame assembly 30 and is configured to guide the barrier panel 54 during movement of siderails 48, 50 between the respective raised and lowered positions. In some embodiments, barrier panel 54 is maintained by the linkage 56 in a substantially vertical orientation during movement of siderails 48, 50 between the respective raised and lowered positions.

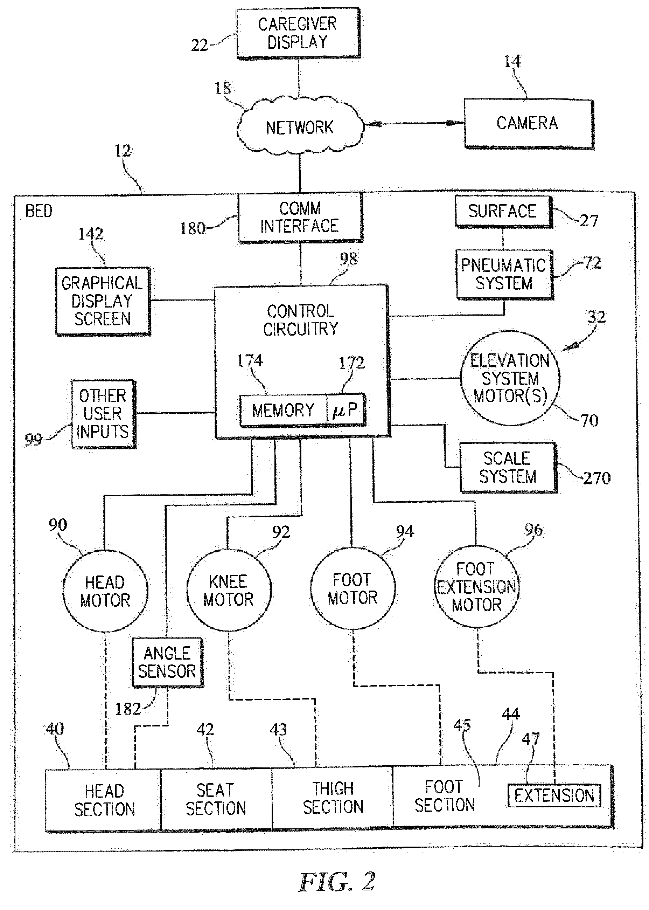

Upper frame assembly 30 includes an upper frame 34 (which in some embodiments includes a lift frame and a weigh frame supported with respect to the lift frame) and a patient support deck 38. Patient support deck 38 is carried by upper frame 34 and engages a bottom surface of a mattress or surface 27. Patient support deck 38 includes a head section 40, a seat section 42, a thigh section 43 and a foot section 44 as shown diagrammatically in FIG. 2. Sections 40, 43, 44 are each movable relative to upper frame 34. For example, head section 40 pivotably raises and lowers relative to seat section 42, whereas foot section 44 pivotably raises and lowers relative to thigh section 43. Additionally, thigh section 43 articulates relative to seat section 42. Also, in some embodiments, foot section 44 is extendable and retractable to change the overall length of foot section 44 and therefore, to change the overall length of deck 38. For example, foot section 44 includes a main portion 45 and an extension 47 in some embodiments as shown diagrammatically in FIG. 2.

In the illustrative embodiment, seat section 42 is fixed in position with respect to upper frame 34 as patient support deck 38 moves between its various patient supporting positions. In other embodiments, seat section 42 also moves relative to upper frame 34, such as by pivoting and/or translating. Of course, in those embodiments in which seat section 42 translates along upper frame 34, the thigh and foot sections 43, 44 also translate along with seat section 42. In some embodiments, bed 12 is able to move to a chair position. As bed 12 moves toward the chair position, foot section 44 lowers relative to thigh section 43 and shortens in length due to retraction of the extension 47 relative to main portion 45. As bed 12 moves away from the chair position, foot section 44 raises relative to thigh section 43 and increases in length due to extension of the extension relative to main portion 45. Thus, in the chair position, head section 40 extends upwardly from upper frame 34 and foot section extends downwardly from thigh section 43.

As shown diagrammatically in FIG. 2, bed 12 includes a head motor or actuator 90 coupled to head section 40, a knee motor or actuator 92 coupled to thigh section 43, a foot motor or actuator 94 coupled to foot section 44, and a foot extension motor or actuator 96 coupled to foot extension 47. Actuators 90, 92, 94, 96 may include, for example, an electric motor of a linear actuator. In those embodiments in which seat section 42 translates along upper frame 30 as mentioned above, a seat motor or actuator (not shown) is also provided. Head actuator 90 is operable to raise and lower head section 40, knee actuator 92 is operable to articulate thigh section 43 relative to seat section 42, foot actuator 94 is operable to raise and lower foot section 44 relative to thigh section 43, and foot extension actuator 96 is operable to extend and retract extension 47 of foot section 44 relative to main portion 45 of foot section 44.

In some embodiments, bed 12 includes a pneumatic system 72 that controls inflation and deflation of various air bladders or cells of surface 27. The pneumatic system 72 is represented in FIG. 2 as a single block but that block 72 is intended to represent one or more air sources (e.g., a fan, a blower, a compressor) and associated valves, manifolds, air passages, air lines or tubes, pressure sensors, and the like, as well as the associated electric circuitry, that are typically included in a pneumatic system for inflating and deflating air bladders of mattresses of hospital beds.

As also shown diagrammatically in FIG. 2, lift system 32 of bed 12 includes one or more elevation system motors or actuators 70, which in some embodiments, comprise linear actuators with electric motors. Thus, motors 70 are sometimes referred to herein as actuators 70. Alternative actuators or motors contemplated by this disclosure include hydraulic cylinders and pneumatic cylinders, for example. The actuators 70 of lift system 32 are operable to raise, lower, and tilt upper frame assembly 30 relative to base 28. In the illustrative embodiment, one of actuators 70 is coupled to, and acts upon, a set of head end lift arms 78 and another of actuators 70 is coupled to, and acts upon, a set of foot end lift arms 80 to accomplish the raising, lowering and tilting functions of upper frame 30 relative to base 28. Arms 78, 80 can be seen in Fig. 1.

In the illustrative example, bed 12 has four foot pedals 84a, 84b, 84c, 84d coupled to base 28 as shown in FIG. 1. Foot pedal 84a is used to raise upper frame assembly 30 relative to base 28, foot pedal 84b is used to lower frame assembly 30 relative to base 28, foot pedal 84c is used to raise head section 40 relative to frame 36, and foot pedal 84d is used to lower head section 40 relative to frame 36. In other embodiments, foot pedals 84a-d are omitted. Pedals having other bed movement functions are also within the scope of this disclosure, such as pedals to move the deck 38 into our out of the chair configuration and/or pedals to move upper frame 34 into or out of the Trendelenburg and reverse Trendelenburg positions.

In some embodiments, one or more of siderails 48, 50 include a user control panel (not shown) coupled to the outward side of the associated barrier panel 54. Such controls panels include various buttons that are known in the art and that are used by a caregiver to control associated functions of bed 12. For example, control panel 66 includes buttons that are used to operate head actuator 90 to raise and lower the head section 40, buttons that are used to operate knee actuator 92 to raise and lower the thigh section 43, and buttons that are used to operate actuators 70 to raise, lower, and tilt upper frame assembly 30 relative to base 28. Such control panels may also include buttons that are used to operate actuator 94 to raise and lower foot section 44 and buttons that are used to operate actuator 96 to extend and retract foot extension 47 relative to main portion 45. In some embodiments, the buttons of the control panels comprise membrane switches, for example.

As shown diagrammatically in FIG. 2, bed 12 includes control circuitry 98 that is electrically coupled to actuators 90, 92, 94, 96 and to actuators 70 of lift system 32. Control circuitry 98 is represented diagrammatically as a single block 98 in FIG. 2, but control circuitry 98 in some embodiments comprises various circuit boards, electronics modules, and the like that are electrically and communicatively interconnected. Control circuitry 98 includes one or more microprocessors 172 or microcontrollers that execute software to perform the various control functions and algorithms of bed 12 as described herein. Thus, circuitry 98 also includes memory 174 for storing software, variables, calculated values, and the like as is well known in the art.

As also shown diagrammatically in FIG. 2, a user inputs block 99 represents the various user inputs such as buttons of control panels and pedals 84a-d, for example, that are used by the caregiver or patient to communicate input signals to control circuitry 98 of bed 12 to command the operation of the various actuators 70, 90, 92, 94, 96 of bed 12, as well as commanding the operation of other functions of bed 12. Bed 12 includes at least one graphical user input or display screen 142 which in some embodiments, is coupled to a respective siderail 48. Display screen 142 is electrically coupled to control circuitry 98 as shown diagrammatically in FIG. 2. In some embodiments, two graphical user interfaces 142 are provided and are coupled to respective siderails 48. Alternatively or additionally, one or more graphical user interfaces are coupled to siderails 50 and/or to one or both of the headboard 46 and footboard 65. Thus, it is contemplated by this disclosure that a graphical user interface 142 may be coupled to any of barriers 65, 46, 48, 50 of bed 12. Alternatively or additionally, graphical user interface 142 is provided on a hand-held device such as a pod or pendant that communicates via a wired or wireless connection with control circuitry 98.

Control circuitry 98 receives user input commands from graphical display screen 142 when display screen 142 is activated. The user input commands control various functions of bed 12 such as controlling the pneumatic system 72 and therefore, the surface functions of surface 22. In some embodiments, the input commands entered on user interface 142 also control the functions of one or more of actuators 70, 90, 92, 94, 96 but this need not be the case. In some embodiments, input commands entered on the user interface 142 also control functions of a scale system 270, which is discussed in more detail below.

Various examples of the various alternative or additional functions of bed 12 that are controlled by display screen 142 in various embodiments can be found in U.S. Patent Application Publication Nos. 2008/0235872 A1, 2008/0172789 A1 and 2012/0089418 A1, each of which is hereby incorporated by reference herein in its entirety to the extent not inconsistent with the present disclosure which shall control as to any inconsistencies. According to this disclosure, control circuitry 98 is configured to deactivate display screen 142 if screen 142 has not been used to control a function of bed 12 within a threshold amount of time, such as 30 seconds to 5 minutes, for example.

In some embodiments, control circuitry 98 of bed 12 communicates with one or more remote computer devices of network 18 via communication infrastructure such as an Ethernet of a healthcare facility in which bed 12 is located and as shown diagrammatically in FIG. 2. Such remote computer devices may be part of an electronic medical records (EMR) system, for example. However, it is within the scope of this disclosure for circuitry 98 of bed 12 to communicate with other computers such as those included as part of a nurse call system, a physician ordering system, an admission/discharge/transfer (ADT) system, or some other system used in a healthcare facility in other embodiments. Network 18 in FIG. 2 is illustrated diagrammatically and is intended to represent all of the hardware and software that comprises a network of a healthcare facility.

In the illustrative embodiment, bed 12 has a communication interface or port 180 which provides bidirectional communication with network 18. A communications link between interface 180 and network 18, in some embodiments, comprises a cable that connects bed 12 to a wall mounted jack that is included as part of a bed interface unit (BIU) or a network interface unit (NIU) 20 of the type shown and described in U.S. Pat. Nos. 7,538,659 and 7,319,386 and in U.S. Patent Application Publication Nos. 2009/0217080 A1, 2009/0212925 A1 and 2009/0212926 A1. As mentioned above, in other embodiments, the communications link between interface 180 and network 18 comprises wireless signals sent between bed 12 and a wireless interface unit of the type shown and described in U.S. Patent Application Publication No. 2007/0210917 A1. Thus, the various communications links disclosed herein comprises one or more wired links and/or wireless links, according to this disclosure.

In some embodiments, operation of any one or more of actuators 70, 90, 92, 94, 96 constitutes a bed event that results in video images captured by camera 14 being recorded and/or transmitted to caregiver display 22. The same goes for operation of pneumatic system 72 to initiate a therapy, for example, such as rotation therapy, percussion therapy, alternating pressure therapy, and the like. Selection of any one or more buttons of user inputs 99, including pedals 84a-84d, or selection of an icon or button display screen 142 also may be considered a bed event that triggers video recording and/or transmission. Furthermore, raising and/or lowering of any of siderails 48, 50 also constitutes a bed event that results in video images captured by camera 14 being recorded and/or transmitted to caregiver display 22 in some embodiments.

Detection by scale system 270 of patient movement by a threshold amount, such as movement toward exiting bed 12, is also a bed event that triggers video recording and/or transmission according to this disclosure. In some embodiments, scale system 270 includes load cells that sense movement of a patient on bed 12. Thus, scale system 270 also serves as a patient position monitoring (PPM) system in some embodiments. See, for example, U.S. Pat. No. 7,253,366 which shows and describes such a scale/PPM system. In some embodiments, other types of weight or position sensors, such as force sensitive resistors (FSR's), capacitive sensors, linear variable displacement transducers (LVDT's), or the like are used in lieu of, or in addition to, the load cells of scale system 270 to provide signals for determining a patient's weight or position.

As shown in Fig. 2, bed 12 includes an angle sensor 182 that senses an angle at which head section 40 is elevated relative to horizontal or relative to another portion of bed 12, such as upper frame 34. This is sometimes referred to as head-of-bed (HOB) angle. It is contemplated by this disclosure that the HOB angle being above or below a threshold angle constitutes a bed event that triggers video recording and/or transmission. For example, doctors sometimes order that the HOB angle be above thirty degrees for a prescribed period of time, such as two to four hours. If head section 40 is moved to a position below the threshold HOB angle, then video recording/transmission is triggered. In some embodiments, the threshold HOB angle is selectable by a user of a computer device of network 18 or by a user of graphical display screen 142 of bed 12. Having the video recording/transmission trigger in response to movement of head section 40 of bed above a threshold angle is also within the scope of this disclosure.

As shown diagrammatically in Fig. 1, system 10 includes a real time location system (RTLS) 110 that determines the location of caregivers throughout a healthcare facility. RTLS systems are well known in the art and are sometimes referred to as locating and tracking systems. In such systems, caregivers wear badges or tags 112 that transmit wireless signals, either periodically or when prompted to do so by components of the RTLS 110. Receivers of RTLS 110 that are mounted at known locations receive the wireless signals from badges 112 and then, computer devices of RTLS 110 determine the location of the caregiver by correlating the badge ID with the receiver ID. Additional details of locating and tracking systems, such as RTLS 100, can be found, for example, in U.S. Pat. Nos. 8,310,364; 8,139,945; 8,082,160; 8,018,584; 7,907,053; 7,734,476; 7,715,387; 7,450,024; 7,567,794; 7,403,111; 7,099,895; 7,053,831; 6,972,683; 6,838,992; 6,825,763; 6,154,139 and 5,455,851.

According to some embodiments of this disclosure, if RTLS 110 indicates that a caregiver is located in the same room at which a bed event occurs, which bed event would otherwise constitute a video recording/transmission trigger, then camera 14 is not turned on and the video feed is not recorded or transmitted to caregiver display 22. The reason being that, since there is a caregiver in the room already, that caregiver is either the reason for the triggering event (e.g., the caregiver is moving a part of the bed 12 or is activating a surface therapy or is helping or watching the patient exiting the bed 12) or is able to supervise the occurrence of the triggering event and maintain the patient's safety. Thus, in some embodiments, the caregiver's presence in a room results in computer device of network 18 suppressing the recording and transmission of the video feed from the camera 14 of the associated room.

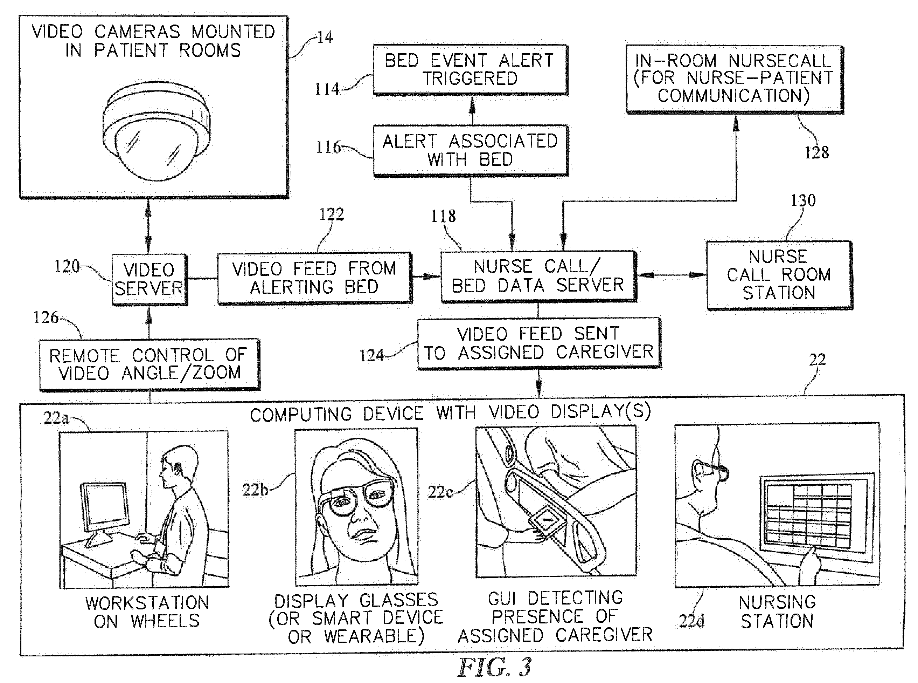

Referring now to Fig. 3, block 14 represents all of the video cameras 14 mounted in patient rooms and block 22 represents the various types of computer devices having caregiver displays 22. In the illustrative example, block 22 indicates that caregiver displays 22 are included in a workstation on wheels 22a (aka a computer on wheels or COW), display glasses 22b (or other smart device or wearable such as a portable phone, wrist watch, wrist band, or the like), a graphical user interface (GUI) 22c of a bed 12, and a nursing station 22d. It is within the scope of this disclosure for a caregiver to be transporting multiple portable computer devices such as two or more of devices 22a, 22b, 22c, 22d. In some embodiments, the video feed associated with a triggering event is transmitted to all such devices being transported by the associated caregiver.

In the case of GUI 22c, it should be appreciated that the bed 12 associated with GUI 22c is located in a different room than the room in which the bed event triggering the video feed is located. Thus, in some embodiments, the caregiver views on GUI 22c located in one room of the healthcare facility, a video feed being captured by a camera 14 in another room of the healthcare facility. Information from the RTLS system 110 is used to determine the location of a caregiver assigned to the patient that is triggering the bed event so that the video feed is routed to the proper GUI 22c near the assigned caregiver.

Still referring to Fig. 3, block 114 represents a bed event or alert being triggered and block 116 represents the alert being associated with a particular bed. That is, a bed ID is transmitted with the information regarding the particular bed event. In the illustrative example, the bed event alert 116 is transmitted to a nurse call or bed data server 118. Based on the bed ID included as part of the alert 116, server 118 is able to determine a location or room ID of the bed 12 that is producing the alert 116. In response to receipt of the alert 116, server 118 communicates with a video server 120 to request a video feed from the camera 14 associated with the room ID. In response, the video server 120 provides the requested video feed from the alerting bed 12 as indicated at block 122. As indicated at block 124, server 118 then transmits the video feed to the computer device 22a, 22b, 22c, 22d having caregiver display 22 of the caregiver assigned to the bed 12 producing the alert 116 or assigned to the room in which the bed 12 producing the 116 alert is located or assigned to the corresponding patient which is associated with the bed 12 producing the alert 116. It will be appreciated that server 118 stores bed-to-room; bed-to-patient; and/or bed-to-patient-to-room association information therein or otherwise has access to some or all of this information, such as by querying RTLS 110.

As further shown in Fig. 3, server 118 is coupled to a nurse call room station 130. Nurse call room stations 130 are included as part of a nurse call system of network 18 and are located in each patient room in some embodiments. Information regarding the bed event 114 triggering the alert 116 is displayed on the room station 130 of the associated room in some embodiments. When a caregiver enters the room to attend to the triggering event, the caregiver selects an icon or button on station 130 to notify server 118 of the caregiver's presence in the patient room. In response to the notification from station 130, server 118 signals server 120 to terminate the video feed 122. This use of station 130 to terminate the video feed 122 is beneficial for embodiments of system 10 that do not include RTLS 110.

According to this disclosure, the computing device 22a, 22b, 22c, 22d having caregiver display 22 is able to function as a remote control of a video angle and/or zoom of camera 14 as indicated at block 126 of Fig. 3. It is contemplated by this disclosure that, in some embodiments, control of camera 14 by the caregiver's computing device in this manner is active while the video feed is being transmitted to the caregiver's computing device and the caregiver is viewing the video feed on display 22. In some embodiments, computing devices 22a, 22b, 22c, 22d are actively usable by caregivers to request that a video feed be provided to display 22 from camera 14 of a particular room even though a triggering event may otherwise not be occurring in the room. Such a request is communicated from the caregiver's computing device to server 118 and then to server 122. In the illustrative example, the use or caregiver display 22 to control angle and/or zoom of camera 14 is communicated to server 120 without involving server 118. For example, a wireless access point of network 18 receives commands relating to zoom and/or angle and transmits it to video server 120. In other embodiments, such commands are routed to server 120 through server 118.

As indicated at block 128 of Fig. 3, in-room nurse call for nurse-to-patient communication is provided in system 10. Such communication is accomplished via a speaker and microphone provided in bed 12 or station 130 or on a pillow speaker unit that is well-known in the art. A speaker that also serves as a microphone is also known in the art and is within the scope of the present disclosure for providing the in-room communication represented by block 128. Thus, server 118 opens an audio channel between computing device 22a, 22b, 22c, 22d, as the case may be, and the speaker/mic of block 128 located in the room at which the triggering event 116 is occurring. This audio channel is opened automatically in some embodiments and/or is opened in response to user inputs by the caregiver on the associated device 22a, 22b, 22c, 22d in some embodiments. So, for example, if the video feed 122 shows that the patient is attempting to get out of bed, the caregiver can provide audio commands to the patient to stay in bed and to otherwise have a conversation with the patient.

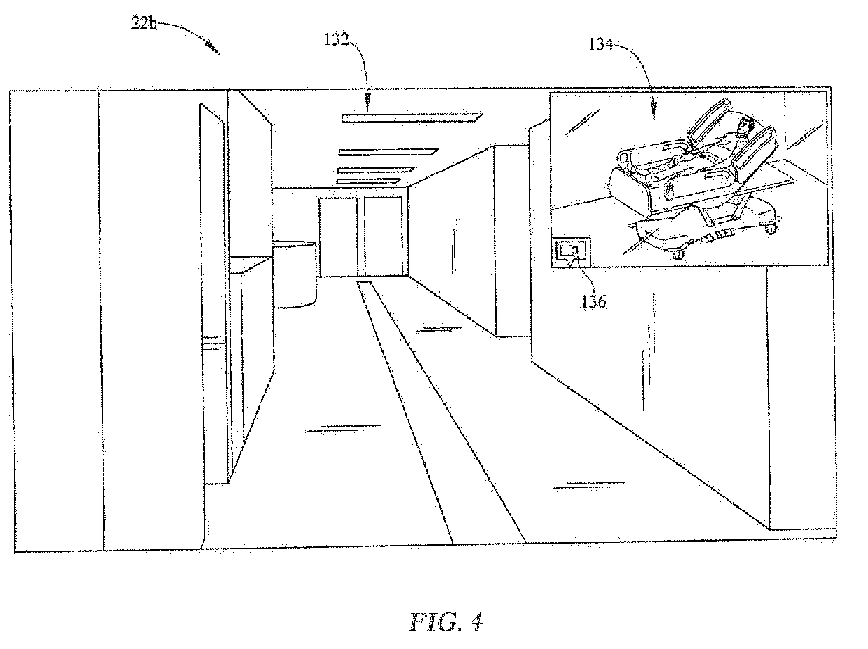

Referring now to Fig. 4, if caregiver display 22 is included as part of display glasses 22b for a particular caregiver, then when the video feed is provided to display glasses 22b, a field of view 132 of the glasses has a video image field 134 that occupies the upper right corner of the field of view 132. The video image captured by the associated camera 14 is what is shown in the video image field 134, while the remainder of field of view 132 is the surrounding environment that is viewed by the caregiver through the glasses 22b. Thus, in the illustrative example, the caregiver is viewing a hallway in field of view 132 and video image field 134 appears to show the patient and bed at which the triggering event has occurred. A video camera icon 136 is provided in the lower left corner of the video image field 134 to indicate that image is a video feed.

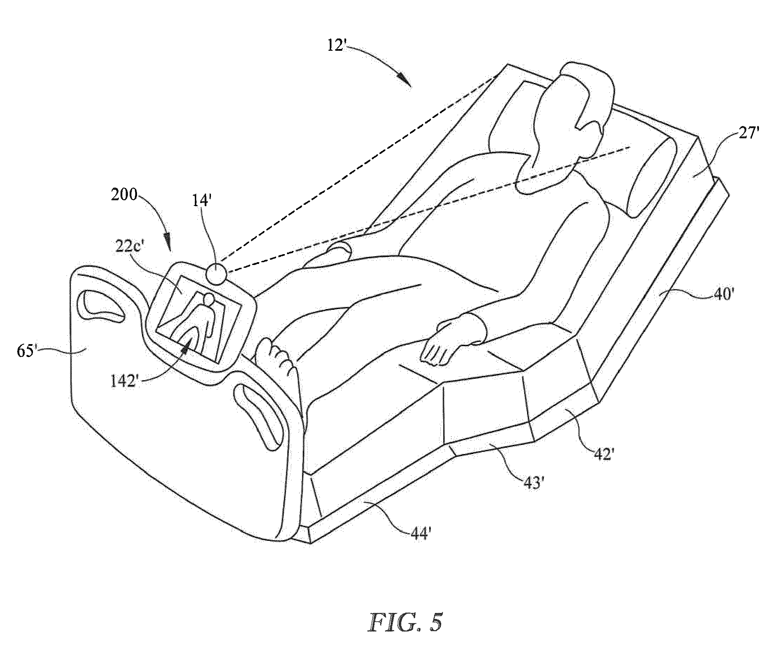

Referring now to Fig. 5, a patient bed 12' has a footboard 65' with a camera module or unit 200 extending upwardly from a central region of a foot board 65'. The camera module 200 has a camera 14' that faces toward a patient on the bed 12' and a GUI display screen 142' which also serves as caregiver display 22c' in the illustrative embodiment. Control circuitry (not shown) of bed 12', which is similar to the circuitry of bed 12 shown diagrammatically in Fig. 2, commands the camera 14' to take a picture of the patient at substantially the same time as a patient weight reading is taken using a weigh scale system (not shown) of the bed 12' and the GUI display screen 142' displays the picture that was taken. In some embodiments, the weight reading and picture are transmitted from the circuitry of the bed 12' for receipt by a remote computer of network 10 such as those mentioned above. In some embodiments, camera 14' captures a video image rather than a picture and the video image is provided to caregiver display 22 of an associated caregiver as a video feed in the same manner as described above. Thus, in some embodiments, camera 14' of bed 12'operates in the same manner as camera 14 within system 10 as described above.

Based on the foregoing, it will be appreciated, for example, that when a bed exit or PPM alarm is triggered (or any other triggering event occurs), a live video feed captured by camera 14 or camera 14' appears substantially immediately on caregiver display 22 of an assigned caregiver. For example, the live video feed appears substantially immediately in field 134 if the assigned caregiver has display glasses 22b. The caregiver then decides whether to intervene such as talking to the patient via in-room communication equipment 128 to ask the patient to stay in bed 12 or to wait for assistance while the caregiver rushes to the bedside. On the other hand, based on the video feed, the caregiver may decide that the patient does not need help. Thus, directly observing the patient via the video feed helps to reduce patient falls by increasing effective interventions while reducing false alarm responses that least to alert fatigue.

Triggering substantially instant remote observation upon a triggering event, such as a bed exit or PPM alarm, combined with audio communication capability leads to increased specificity by recognizing which patients do not require assistance (e.g., the patient is not actually exiting the bed 12), leads to increased sensitivity by promoting more regular use of bed monitoring systems such as the bed exit/PPM system, and lead to enhanced interventions by allowing more sensitive PPM settings to be selected for more early intervention, by allowing caregivers to respond more quickly to patients that do need immediate help, and by communicating with the patient while en route to the patient's room.

According to some embodiments of this disclosure, a caregiver is able to retrieve past video recordings or past pictures for viewing on caregiver display 22 such as displays 22a, 22b, 22c, 22c', 22d. The past video recordings or past pictures are stored in one or more computer devices of network 18 such as one or both of servers 118, 120. The caregiver uses the computer device associated with display 22 to request a particular past video recording or past picture and the computer device storing the past video recording or picture responds by transmitting the requested video or picture to the caregiver's device. Such past videos or pictures are useful, for example, in connection with weighing a patient on the patient bed 12, 12' or in connection with zeroing weigh scale system 270 of patient bed 12, 12'. For example, the caregiver compares the past video or picture with the current state of the patient bed 12, 12' to check for any changes in equipment, sheets, pillows, etc. on the bed 12, 12'. If there are changes as compared to the past state of the bed 12, 12', the caregiver may take steps to re-zero the weigh scale system 270 so that subsequent weight readings of the associated patient are accurate.

Although certain illustrative embodiments have been described in detail above, variations and modifications are possible.

## Claims

1. A system comprising a patient bed, and a camera system that automatically, in response to an occurrence of a bed event, records a video feed of a patient on the bed for a period of time and/or or transmits a video feed of the patient for a period of time.

2. The system of claim 1, wherein the bed event comprises a weight reading being taken.

3. The system of claim 2, wherein the weight reading and the video feed are transmitted from the bed to a remote computer, together, optionally, with a picture of the patient taken by the camera system.

4. The system of any preceding claim, wherein the bed event comprises movement of the patient on the bed.

5. The system of claim 4, wherein the movement of the patient on the bed comprises a threshold amount of movement that is sufficient to trigger a patient position monitoring alarm and/or a bed exit alarm of the patient bed.

6. The system of any preceding claim, wherein the camera system comprises a camera and at least a first computer device remote from the camera.

7. The system of any preceding claim further comprising a first computer device remote from the patient bed, and a portable computer device transportable by a caregiver, the video feed being sent to the portable computer device by the first computer device in response to receipt of the video feed from the camera system.

8. The system of claim 7, wherein the portable computer device comprises a workstation on wheels.

9. The system of claim 7, wherein the portable computer device comprises a wearable device.

10. The system of claim 9, wherein the wearable device comprises eye glasses with video display capability in a field of view of the caregiver or wherein the wearable device is configured to be worn on the caregiver's wrist.

11. The system of claim 7, wherein the portable computer device comprises a handheld phone.

12. The system of any one of claims 6 to 11, wherein the video feed is transmitted by the first computer device to a display of a patient bed that is near the caregiver, or the video feed is transmitted to a display mounted to a wall of a room in which the caregiver is located, or the video feed is transmitted to a nurse station computer that is located at a nursing station.

13. The system of any one of claims 7 to 12, wherein, in response to the video feed being sent to the portable computer device, an audio channel is opened between the portable computer device and a speaker in the patient's room so that the caregiver is able to talk to the patient.

14. The system of any one of claims 6 to 13 , wherein the first computer device records the video feed for a period of time.

15. The system of any one of claims 6 to 14, further comprising a second computer device remote from the patient bed, the first computer device transmitting the video feed to the second computer device for recording and storage by the second computer device.
